# EUROPÄISCHE PATENTSCHRIFT

(11) **EP 2 864 242 B1**
(45) Veröffentlichungstag und Bekanntmachung des Hinweises auf die Patenterteilung: **28.11.2018**
(21) Anmeldenummer: 13723409.2
(22) Anmeldetag: 08.05.2013
(51) Int. Cl.: C01B 3/12, C01B 3/52, C01B 3/50, C01B 3/56, F25J 3/02

(54) **VERFAHREN ZUR HERSTELLUNG VON CO, H2 UND METHANOL-SYNTHESEGAS AUS EINEM SYNTHESEGAS, INSBESONDERE AUS ACETYLEN-OFFGAS**
METHOD FOR PRODUCING CO, H2 AND METHANOL SYNTHESIS GAS FROM A SYNTHESIS GAS, IN PARTICULAR FROM ACETYLENE OFF GAS
PROCÉDÉ DE PRODUCTION DE CO, DE H2 ET DE GAZ DE SYNTHÈSE DE MÉTHANOL À PARTIR D'UN GAZ DE SYNTHÈSE, EN PARTICULIER À PARTIR D'UN EFFLUENT GAZEUX ACÉTYLÉNIQUE

(30) Priorität: 24.05.2012 DE 102012010312
(43) Veröffentlichungstag der Anmeldung: 29.04.2015
(73) Patentinhaber: Linde Aktiengesellschaft, 80331 München (DE)
(72) Erfinder: TROTT, Thomas, 80686 München (DE)
(86) Internationale Anmeldenummer: PCT/EP2013/001382
(87) Internationale Veröffentlichungsnummer: WO 2013/174480

(56) Entgegenhaltungen:
- EP-A1- 1 729 077
- EP-A1- 1 918 352
- EP-A2- 0 563 702
- EP-A2- 1 975 123
- WO-A2-2009/019497

## Beschreibung

Die Erfindung betrifft ein Verfahren zur Herstellung eines Methanol-Synthesegases, eines H₂-Produktes und eines CO-Produktes aus einem H₂- und CO-haltigen Synthesegas, insbesondere einem Actetylen-Offgas.

Verfahren zur Herstellung der oben genannten Produkte aus einem Synthesegas sind aus dem Stand der Technik bekannt, siehe z. B. EP 1 975 123 A2. Hierbei erweist es sich jedoch als Herausforderung die drei genannten Produkte in stark schwankenden Mengen im Rahmen eines möglichst ökonomischen Verfahrens aus einem ggf. stark verunreinigten, H₂ und CO aufweisendem Synthesegas zu erzeugen.

Dieses Problem wird durch ein Verfahren mit den Merkmalen des Anspruchs 1 gelöst. Weitere Ausführungen sind in den Unteransprüchen offenbart. Danach wird bei dem erfindungsgemäßen Verfahren ein Synthesegasstrom bereitgestellt, in einen ersten und einen zweiten Synthesegasteilstrom aufgeteilt, wobei lediglich im ersten Synthesegasteilstrom enthaltenes CO mit dem ersten Synthesegasteilstrom zugemischten Wasserdampf zu CO₂ und H₂ konvertiert wird (Wassergas-Shift-Reaktion), wobei der erste konvertierte Synthesegasteilstrom und ein Teil des zweiten unkonvertierten Synthesegasteilstromes jeweils zum Auswaschen von CO₂ mit einem aminhaltigen Waschmittel (z.B. MDEA) zwei getrennten Waschkolonnen zugeführt werden, wobei das Waschmittel in einer gemeinsamen Kolonne regeneriert wird, wobei der Methanol-Synthesegasproduktstrom aus einem Teil des gewaschenen, konvertierten ersten Synthesegasteilstromes und/oder dem anderen Teil des unkonvertierten zweiten Synthesegasstromes sowie ggf. zwei weiteren Strömen (DWA-Restgas und Roh-H₂ aus einer Cold Box, siehe unten) gemischt wird, derart, dass ein für die Methanol-Synthese notwendiges Verhältnis von (H₂-CO₂)/(CO+CO₂) eingestellt wird, und zwar im Bereich von 2,0 bis 2,1, wobei des Weiteren der gewaschene eine Teil des zweiten nicht-konvertierten Synthesegasteilstromes zur Herstellung des CO-Produktstromes und des H₂-Produktstromes verwendet wird, und wobei der andere Teil (soweit vorhanden) des gewaschenen konvertierten ersten Synthesegasteilstromes zur Herstellung des H₂-Produktstromes verwendet wird. Bevorzugt wird der Synthesegasstrom (Einsatzgas) in einem ersten Prozessschritt verdichtet, um insbesondere die gewünschten Produktdrücke (H₂ und Methanol-Syngas) ohne weitere Verdichtung erreichen zu können. Hierdurch kann des Weiteren das effektive Gasvolumen und damit die Dimensionen der verwendeten Anlagenkomponenten reduziert werden. Weiterhin muss hierdurch der für die CO-Konvertierung verwendete Dampf nicht übermäßig entspannt werden. Zudem wird ein besseres Absorptionsverhalten in der CO₂-Wäsche erreicht, und eine nachfolgende kryogene Gaszerlegung kommt mit einer geringeren CO-Kreislaufmenge aus, was Durchsatz und Verbrauch beim CO-Verdichter (Verdichtung des CO-Produktstromes und des CO-Kreislaufstromes) reduziert. Schließlich wird durch die eingangs vorgesehene Verdichtung des Einsatzes bei einer Druckwechseladsorption (DWA) die H₂-Ausbeute erhöht und damit die zu verdichtende Restgasmenge reduziert.

Besonders bevorzugt wird als Synthesegasstrom (Einsatzstrom) ein bislang kaum zur weiteren Wertstoffgewinnung genutztes Actetylen-Offgas herangezogen, das üblicherweise bei der Acetylen-Herstellung in großen Mengen als preiswertes Nebenprodukt anfällt.

Eine typische Spezifikation eines solchen Offgases (AOG) in mol% sieht folgendermaßen aus:

| Bestandteil | mol% |
|---|---|
| H₂ | 59 bis 64 |
| CO | 29 bis 34 |
| CO₂ | 3,3 bis 4,1 |
| CH₄ | 1,5 bis 3,5 |
| N₂+Ar+He | 0,3 bis 1,0 |
| O₂ | 0,1 bis 0,6 |
| C₂H₆ | 0,001 bis 0,1 |
| C₂H₄ | 0,2 bis 0,6 |
| C₃H₄ | 0,003 bis 0,03 |
| Gesamt S | <0,1 mg/Nm3 |
| NMP | Spuren |
| Temperatur | 15°C bis 40°C |
| Druck | 9 bar bis12 bar |

Das erfindungsgemäße Verfahren zeichnet sich insbesondere durch die Möglichkeit aus, sowohl die Einstellung der jeweils benötigten CO- und H₂-Produktmenge als auch - im Zusammenhang hiermit - die Einstellung des (H₂-CO₂)/(CO+CO₂)-Verhältnisses im Methanol-Synthesegasproduktstrom, das für die Methanol-Synthese (CO+2H₂ ↔ CH₃OH) in einem Bereich von 2,0 bis 2,1 liegt, zu realisieren.
Die vorgenannte Einstellung kann insbesondere durch entsprechendes Aufteilen des Synthesegasstromes in die beiden Synthesegasteilströme sowie deren Aufteilung auf den herzustellenden Methanol-Synthesegasproduktstrom und den herzustellenden Wasserstoff- bzw. CO-Produktstrom vorgenommen werden.

Hierzu kann z.B. zur Einstellung der CO-Produktmenge der zweite nicht konvertierte Synthesegasteilstrom über ein erstes Ventil der besagten Aminwäsche zugeführt werden, so dass bei entsprechender Stellung des ersten Ventils ein Teil des zweiten Synthesegasteilstromes über das besagte erste Ventil in die Aminwäsche gelangt und für die Herstellung des CO-Produktstromes bzw. H₂-Produktstromes zur Verfügung steht und der andere Teil stromauf des ersten Ventils abgezweigt und in den Methanol-Synthesegasproduktstrom gegeben wird.

Weiterhin kann der konvertierte gewaschene erste Synthesegasteilstrom zur Einstellung der H₂-Produktmenge über ein zweites Ventil zur Druckwechseladsorptionseinheit (DWA) gegeben werden (siehe unten), wobei bei entsprechender Stellung des zweiten Ventils ein Teil des gewaschenen ersten konvertierten Synthesegasteilstromes stromauf des zweiten Ventils abgezweigt und in den Methanol-Synthesegasproduktstrom gegeben wird, und wobei der andere Teil des gewaschenen ersten Synthesegasteilstromes über das besagte zweite Ventil in die Druckwechseladsorptionseinheit gelangt und zur Herstellung des H₂-Produktstromes zur Verfügung steht.

Das sich hierbei im Methanol-Synthesegasproduktstrom einstellende (H₂- CO₂)/(CO+CO₂)-Verhältnis wird vorzugsweise gemessen (Istwert), wobei der erste Synthesegasteilstrom über ein drittes Ventil in die oben beschriebene CO-Konvertierung gegeben wird und das dritte Ventil so geregelt wird, dass die entsprechende Konvertierung von CO und H₂O in H₂ und CO₂ das besagte Verhältnis auf den vordefinierten Referenzwert im Bereich von 2,0 bis 2,1 regelt. Ist z.B. der H₂-Anteil zu gering, wird der erste Synthesegasteilstrom durch entsprechendes Einstellen des dritten Ventils vergrößert, so dass pro Zeiteinheit mehr CO und H₂O in H₂ und CO₂ umgesetzt wird. Ist der H₂-Anteil zu groß wird der erste Synthesegasteilstrom durch entsprechendes Einstellen des dritten Ventils verringert.
Zusammenfassend wird die Konvertierung bei vorgegebener CO-Produktmenge (erstes Ventil) so durchgeführt, dass einerseits die gewünschte H₂-Produktmenge (zweites Ventil) resultiert und andererseits das besagte Verhältnis im gemischten Methanol-Synthesegasproduktstrom einen für die Methanol-Synthese tauglichen Wert im Bereich von 2,0 bis 2,1 annimmt.
Für den Fall, dass als Einsatz ein Acetylen-Offgasstrom verwendet wird, werden bevorzugt darin enthaltene, ungesättigte Kohlenwasserstoffe vor dem Aufteilen des Synthesegasstromes in einer katalytischen Reinigungseinheit zu gesättigten Kohlenwasserstoffen hydriert, wobei C₂H₂ und/oder C₂H₄ zu C₂H₆ bzw. C₃H₄ und/oder C₃H₆ zu C₃H₈ hydriert wird. Des Weiteren wird im Acetylen-Offgasstrom enthaltener Sauerstoff mit ebenfalls darin enthaltenem H₂ und CO zu H₂O und CO₂ umgesetzt. Zusätzlich werden Schwefel- und NMP-Spuren (N-Methyl-Pyrrolidon) aus dem Acetylen-Offgasstrom in der o. g. katalytischen Reinigungseinheit zurückgehalten.
Vorzugsweise wird der besagte eine Teil des zweiten Synthesegasteilstroms, der insbesondere zur Erzeugung des CO-Produktes bzw. des H2-Produktes dient, nach dem Auswaschen von CO₂ zum Trocknen (Entfernen von Wasser) und zur Entfernung von Rest-CO₂ einer Temperaturwechseladsorption in einer entsprechenden Temperaturwechseladsorptionseinheit unterzogen, wobei zumindest ein Adsorber im besagten Teil des zweiten Synthesegasteilstroms enthaltenes H₂O und CO₂ bei niedrigen Temperaturen adsorbiert und sodann der mit den Komponenten beladene mindestens eine Adsorber bei vergleichsweise höheren Temperaturen durch Spülen mit einem Rohwasserstoffstrom regeneriert wird, wobei dem Rohwasserstoffstrom ein Teil des durch die besagte Temperaturwechseladsorption getrockneten und von CO₂ befreiten Teils des unkonvertierten zweiten Synthesegasteilstroms zugemischt wird.

Der solchermaßen getrocknete und von CO₂ befreite Teil des unkonvertierten zweiten Synthesegasteilstromes wird hiernach in einer Cold Box zumindest in den CO-Produktstrom und einen Rohwasserstoffstrom sowie einen Restgasstrom getrennt (partielle Kondensation oder Methanwäsche), wobei der CO-Produktstrom verdichtet wird, und wobei zumindest ein verdichteter Teilstrom des CO-Produktstroms zur Erzeugung der für die besagte Trennung benötigten Kälte und/oder Wärme verwendet wird (CO-Kreislaufströme). Zur Erzeugung von Kälte wird hierbei insbesondere ein CO-Teilstrom in einem CO-Expander entspannt. Der bei der Trennung entstehende Restgasstrom wird an einer Anlagengrenze abgegeben und/oder verfeuert.
Weiterhin wird der oben erwähnte Rohwasserstoffstrom zum Regenerieren/Spülen des mindestens einen Adsorbers der Temperaturwechseladsorptionseinheit vorzugsweise vom in der Cold Box erzeugten Rohwasserstoffstrom abgespalten und nach dem Regenerieren/Spülen wieder in den Rohwasserstoffstrom zurückgeführt.
Zur Erzeugung des H₂-Produktstromes wird nun bevorzugt der Rohwasserstoffstrom und der andere Teil des gewaschenen konvertierten ersten Synthesegasteilstromes zu einem wasserstoffreichen DWA-Einsatzstoffstrom vermischt, der zur weiteren Reinigung einer Druckwechseladsorption (DWA) unterzogen wird. Hierbei wird der DWA-Einsatzstoffstrom unter hohem Druck durch zumindest einen Adsorber gegeben, wobei darin enthaltener Wasserstoff den mindestens einen Adsorber passiert und den H₂-Produktstrom bildet. Die im DWA-Einsatzstoffstrom enthaltenen schwereren Komponenten, wie z.B. CO, werden am mindestens einen Adsorber adsorbiert. Ist der mindestens eine Adsorber vollständig beladen, werden die adsorbierten Komponenten bei niedrigerem Druck desorbiert und der mindestens eine Adsorber insbesondere mit einem aus einem Teilstrom des erzeugten H₂-Produktstromes gebildeten Spülgas gespült. Ein hierbei gebildeter, die desorbierten Komponenten sowie das Spülgas enthaltener Restgastrom wird sodann verdichtet und kann als weitere Komponente dem Methanol-Synthesegasproduktstrom zugegeben werden.
Bei Bedarf, insbesondere bei einem vordefinierbar kleinen CO-Produktstrom und einem vergleichsweise großen H₂-Produktstrom, kann ein Teil des gewaschenen Teils des unkonvertierten zweiten Synthesegasteilstromes dem DWA-Einsatzstoffstrom zugemischt werden. Weiterhin kann bei Bedarf, insbesondere bei einem vordefinierbar kleinen H₂-Produktstrom und einem vergleichsweise großen CO-Produktstrom ein Teil des Rohwasserstoffstromes in den Methanol-Synthesegasproduktstrom gegeben werden.

Weiterhin wird eine Anlage zur Herstellung eines H₂-Produktstromes, eines CO-Produktstromes und eines Methanol-Synthesegasproduktstromes aus einem H₂- und CO- enthaltenden Synthesegasstrom (z.B. Acetylenoffgas) beschrieben.

Die Anlage weist vorzugsweise einen Verdichter auf, der dazu ausgebildet ist, den Synthesegasstrom (Einsatzstrom) zu verdichten (siehe oben), wobei der Verdichter vorzugsweise mit einer stromab des Verdichters vorgesehen Reinigungseinheit verbunden ist, die zum katalytischen Hydrieren von im (verdichteten) Synthesegasstrom enthaltenen ungesättigten Kohlenwasserstoffen sowie zur Sauerstoffentfernung ausgebildet ist.
Die besagte Reinigungseinheit ist bevorzugt über ein drittes Ventil mit einem Wassergas-Shift-Reaktor verbunden, so dass ein erster Synthesegasteilstrom über das dritte Ventil in den Wassergas-Shift-Reaktor einspeisbar ist, der zum Konvertieren von im ersten Synthesegasteilstrom enthaltenen CO mit H₂O (Dampf) zu H₂ und CO₂ ausgebildet ist, wobei jene Konvertierungseinheit (Wassergas-Shift-Reaktor) weiterhin mit einer Aminwascheinheit verbunden ist, die wiederum über ein zweites Ventil mit einer Rohwasserstoffzuleitung zu einer Druckwechselabsorptionseinheit (DWA) sowie mit einer Ableitung für den Methanol-Synthesegasproduktstrom verbunden ist, so dass der erste Synthesegasteilstrom vom Wassergas-Shift-Reaktor in die Aminwascheinheit und ein Teil des ersten Synthesegasteilstroms als ein Bestandteil des zu erzeugenden Methanol-Synthesegasproduktstromes in die besagte Ableitung und - je nach Einstellung des zweiten Ventils - der andere Teil des ersten Synthesegasteilstroms über das zweite Ventil in die Rohwasserstoffzuleitung der DWA einspeisbar ist.
Weiterhin ist die Reinigungseinheit vorzugsweise über ein erstes Ventil mit der Aminwascheinheit verbunden, so dass ein Teil des zweiten Synthesegasteilstroms über das erste Ventil in die Aminwascheinheit einspeisbar ist, wobei stromauf des ersten Ventils die besagte Ableitung abzweigt, so dass - je nach Einstellung des ersten Ventils - der andere Teil des zweiten Synthesegasstromes als ein weiterer Bestandteil des Methanol-Synthesegasproduktstromes in jene Ableitung einspeisbar ist.

An der Ableitung (MeOH-Syngas-Leitung) ist - nach Zumischung aller Teilströme - bevorzugt ein Sensor zum Erfassen des (H₂-CO₂)/(CO+CO₂)-Verhältnisses des aus dem einen Teil des (konvertierten) ersten Synthesegasteilstromes, dem anderen Teil des (nicht-konvertierten) zweiten Synthesegasteilstromes sowie ggf. des DWA-Restgases und einem Teil des Rohwasserstoffs aus der Cold Box gemischten Methanol-Synthesegasproduktstromes vorgesehen, wobei zum Regeln des dritten Ventils eine Regeleinheit vorgesehen ist, die dazu ausgebildet ist, jenes Verhältnis (Istwert) durch entsprechendes Einstellen des dritten Ventils auf einen vorgegeben Referenzwert (Sollwert) im Bereich von 2,0 bis 2,1 zu regeln (siehe oben).

Die Aminwascheinheit ist bevorzugt zum separaten Waschen des konvertierten ersten Synthesegasteilstromes sowie des einen Teils des unkonvertierten zweiten Synthesegasteilstromes ausgebildet, um darin enthaltenes CO₂ zu entfernen bzw. zu reduzieren, wobei die Aminwascheinheit zum Regenerieren des jeweils verwendeten, mit CO₂ beladenen Waschmittels vorzugsweise eine gemeinsame Kolonne aufweist, die zum Regenerieren beider Waschmittel ausgebildet ist. Die Aminwascheinheit ist des Weiteren über eine erste Leitung mit einer Temperaturwechseladsorptionseinheit zum Trocknen des gewaschenen einen Teils des unkonvertierten zweiten Synthesegasteilstroms und zur Entfernung von noch darin befindlichen CO₂ verbunden, wobei insbesondere die Temperaturwechseladsorptionseinheit dazu ausgebildet ist, im besagten Teil des zweiten Synthesegasteilstroms enthaltenes H₂O und CO₂ bei niedrigen Temperaturen an zumindest einem Adsorber zu adsorbieren und den mit den besagten Komponenten beladenen mindestens einen Adsorber sodann bei vergleichsweise höheren Temperaturen durch Spülen mit einem Rohwasserstoffstrom zu regenerieren.

Die Temperaturwechseladsorptionseinheit ist vorzugsweise über eine zweite Leitung mit einer kryogenen Trenneinheit (Cold Box) verbunden, so dass der getrocknete und von CO₂ befreite eine Teil des unkonvertierten zweiten Synthesegasteilstroms über jene zweite Leitung in die Cold Box einspeisbar ist, die dazu ausgebildet ist, den getrockneten und von CO₂ befreiten Teil des unkonvertierten zweiten Synthesegasteilstroms in den zu erzeugenden CO-Produktstrom, einen Rohwasserstoffstrom sowie insbesondere einen Restgasstrom zu trennen (siehe oben). Zum Verdichten des CO-Produktstromes auf den gewünschten Produktdruck ist die Cold Box mit einem CO-Verdichter verbunden, der zusätzlich dazu ausgebildet ist, zumindest einen CO-Teilstrom zur Kälte- und/oder Wärmeproduktion auf den gewünschten CO-Kreislaufdruck zu verdichten (auf ca. 30 bar bis 40 bar) und in die Cold Box zurückzuführen. Weiterhin ist die Anlage zur Produktion der für die kryogene Trennung benötigten Kälte insbesondere dazu eingerichtet, zumindest einen der vorgenannten CO-Teilströme des CO-Kreislaufstromes in einem CO-Expander zu entspannen (auf ca. 15 bar bis 20 bar).

Die Cold Box ist des Weiteren bevorzugt über eine Regenerier-/Spülgaszuleitung mit der Temperaturwechseladsorptionseinheit verbunden, die zum Einspeisen des zum Regenerieren/Spülen verwendeten Rohwasserstoffstromes aus der Cold Box in die Temperaturwechseladsorptionseinheit ausgebildet ist. Weiterhin ist eine erste, mit einem Ventil versehene Überbrückungsleitung (Bypass) vorgesehen, die die zweite Leitung mit der Regenerier-/Spülgaszuleitung stromauf der Temperaturwechseladsorptionseinheit verbindet, so dass bei Bedarf, insbesondere im Teillastfall, dem zum Regenerieren/Spülen verwendeten Rohwasserstoffstrom ein Teil des durch die besagte Temperaturwechseladsorption getrockneten und von CO₂ befreiten Teils des zweiten (nicht-konvertierten) Synthesegasteilstroms über die erste Überbrückungsleitung zumischbar ist. Weiterhin ist eine Regenerier-/Spülgasrückleitung vorgesehen, die die Temperaturwechseladsorptionseinheit mit der Wasserstoffzuleitung der Druckwechseladsorptionseinheit verbindet, so dass beladenes Regenerier-/Spülgas (Rohwasserstoffstrom) in die Rohwasserstoffzuleitung zur Druckwechseladsorptionseinheit eingespeist werden kann.

Weiterhin ist bevorzugt eine zweite, mit einem Ventil versehene Überbrückungsleitung (Bypass b) vorgesehen, die die erste Leitung (zwischen der Aminwascheinheit und der Temperaturwechseladsorptionseinheit) mit der Rohwasserstoffzuleitung der Druckwechseladsorptionseinheit verbindet, so dass bei Bedarf ein Teil des gewaschenen Teils des unkonvertierten zweiten Synthesegasteilstromes in die Druckwechseladsorptionseinheit einspeisbar ist. Weiterhin ist eine dritte, mit einem Ventil versehene Überbrückungsleitung (Bypass) vorgesehen, die die Rohwasserstoffzuleitung der Druckwechseladsorptionseinheit mit der besagten Ableitung zum MeOH-Syngas verbindet, so dass bei Bedarf ein in der Rohwasserstoffzuleitung der Druckwechseladsorptionseinheit geführter DWA-Einsatzstoffstrom (Rohwasserstoff aus der Cold Box, ggf. konvertiertes und nichtkonvertiertes Synthesegas sowie Regenerier-/Spülgas aus der TWA) in die Ableitung eingespeist und somit dem Methanol-Synthesegasproduktstrom zugegeben werden kann.

Die Druckwechseladsorptionseinheit ist im Einzelnen bevorzugt dazu ausgebildet, den in der Rohwasserstoffzuleitung geführten (wasserstoffreichen) DWA-Einsatzstoffstrom unter hohem Druck durch zumindest einen Adsorber zu geben, wobei Wasserstoff den mindestens einen Adsorber passiert und den besagten H₂-Produktstrom bildet, und um dabei im DWA-Einsatzstoffstrom enthaltene schwerere Komponenten, insbesondere CO, am mindestens einen Adsorber zu adsorbieren, wobei die Druckwechseladsorptionseinheit des Weiteren dazu ausgebildet ist, die am mindestens einen Adsorber adsorbierten Komponenten bei niedrigerem Druck zu desorbieren und insbesondere mit einem aus einem Teilstrom des erzeugten H₂-Produktstromes gebildeten Spülgas zu spülen. Die Druckwechseladsorptionseinheit ist insbesondere mit einem Restgasverdichter verbunden, der wiederum mit der besagten Ableitung verbunden ist, so dass ein bei der Druckwechseladsorption erzeugter, die desorbierten Komponenten sowie das Spülgas enthaltener Restgastrom in dem Restgasverdichter verdichtet und sodann dem Methanol-Synthesegasstrom zugegeben werden kann.

Weitere Einzelheiten und Vorteile der Erfindung sollen durch die nachfolgende Figurenbeschreibung eines Ausführungsbeispiels anhand der Figur erläutert werden.

Es zeigt:

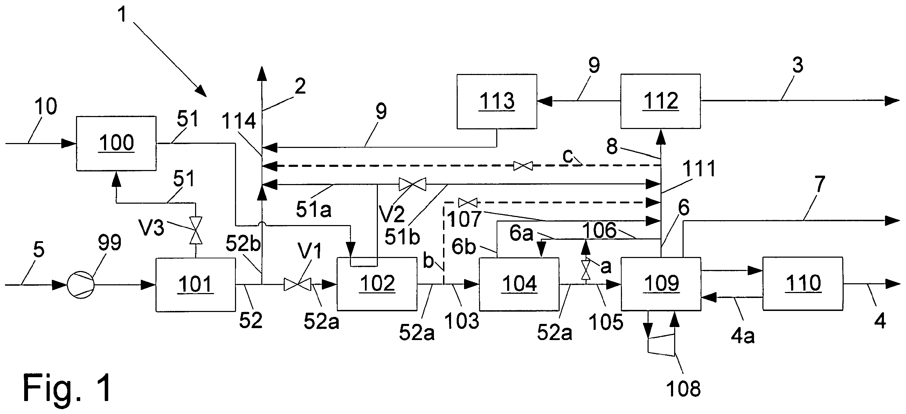

- Fig. 1: ein erfindungsgemäßes Verfahren zur Herstellung eines H₂-, CO- und Methanol-Synthesegasproduktstromes.

Figur 1 zeigt eine schematische Darstellung eines Verfahrens bzw. einer Anlage 1 zum Erzeugen eines H₂-Produktstromes 3, eines CO-Produktstromes 4 sowie eines Methanol-Synthesegasproduktstromes 2, der eine für die Methanolsynthese geeignete Zusammensetzung aufweist. Als eingesetztes Synthesegas 5 wird bevorzugt ein sogenanntes Acetylen-Offgas (AOG) 5 verwendet, bei dem es sich um ein Nebenprodukt bei der Acetylen-Produktion handelt, das in vergleichsweise großen Mengen anfällt.

Das erfindungsgemäße Verfahren ist allerdings nicht auf AOG als Einsatzstoff beschränkt, sondern kann auch für andere Synthesegase 5 ähnlicher Zusammensetzung (v.a. ähnlichem H₂/CO-Verhältnis) mit Sauerstoff und/oder ungesättigten Kohlenwasserstoffen zur Herstellung der drei beschriebenen Produkte 2, 3, 4 angewendet werden. Der beschriebene Prozess ist auch nicht auf den angegebenen Einsatzdruck gemäß obiger Tabelle beschränkt.

In einem ersten Prozessschritt wird der Synthesegasstrom (Acetylen-Offgasstrom oder AOG) 5 zunächst verdichtet 99 und sodann in einer Reinigungseinheit 101 einer katalytischen Reinigung unterzogen, bei der insbesondere in einer 2-stufigen Katalyse die im Synthesegasstrom 5 enthaltenen ungesättigten Kohlenwasserstoffe hydriert werden (C₂H₂ und C₂H₄ zu C₂H₆ bzw. C₃H₄ und C₃H₆ zu C₃H₈) und der enthaltene Sauerstoff mit H₂ bzw. CO zu H₂O bzw. CO₂ umgesetzt wird.

Damit werden sowohl das Ausfrieren ungesättigter Kohlenwasserstoffe in einer nachfolgenden kryogenen Trennung (Cold Box 109), wie auch das Anreichern von explosiven Komponenten wie O₂ und Acetylen (C₂H₂) in allen nachfolgenden Anlagenteilen verhindert. Zusätzlich werden vorzugsweise Schwefel- und NMP-Spuren (N-Methyl-Pyrrolidon) aus dem AOG 5 entfernt.

Nach der vorbeschriebenen initialen Reinigung des Synthesegasstromes (AOG) 5 wird dieser in einen ersten und einen zweiten Synthesegasteilstrom 51, 52 aufgeteilt, wobei der erste (noch warme) Synthesegasteilstrom 51 zusammen mit Hochdruck-Dampf einem Wassergas-Shift-Reaktor 100 zugeführt wird, um durch Konvertierung von CO+H₂O zu CO₂+H₂ bei vorgegebener CO-Produktmenge (erstes Ventil V1) die geforderte H₂-Produktmenge zu erzeugen (zweites Ventil V2) und um das für die Methanol-Synthese notwendige Verhältnis (H₂-CO₂)/(CO+CO₂) im Bereich vorzugsweise 2,0 bis 2,1 im gemischten Methanol-Synthesegasproduktstrom 2 (drittes Ventil V3) einzustellen.

Dabei wird im Einzelnen der zweite Synthesegasteilstrom 52 über das erste Ventil V1 der CO2-Wascheinheit (z.B. Aminwäsche) 102 zugeführt, so dass bei entsprechender Stellung des ersten Ventils V1 ein Teil 52a des zweiten (nicht-konvertierten) Synthesegasteilstromes 52 über das besagte erste Ventil V1 in die Aminwascheinheit 102 eingespeist wird, wobei der andere Teil 52b stromauf des ersten Ventils V1 in eine Ableitung 114 für den Methanol-Synthesegasproduktstrom 2 gegeben wird.

Der konvertierte (gewaschene) erste Synthesegasteilstrom 51 wird zur Einstellung der H₂-Produktmenge über ein zweites Ventil V2 in eine Rohwasserstoffzuleitung 111 einer Druckwechseladsorptionseinheit (DWA) 112 gegeben, wobei bei entsprechender Stellung des zweiten Ventils V2 ein Teil 51a des gewaschenen ersten (konvertierten) Synthesegasteilstromes 51 stromauf des zweiten Ventils V2 in die besagte Ableitung 114 abgezweigt und somit in den Methanol-Synthesegasproduktstrom 2 gegeben wird, und wobei der andere Teil 51b des gewaschenen, konvertierten ersten Synthesegasteilstromes 51 über das besagte zweite Ventil V2 in die Rohwasserstoffzuleitung 111 zur Druckwechseladsorptionseinheit 112 gelangt. Das sich in der Ableitung 114 einstellende (H₂-CO₂)/(CO+CO₂)-Verhältnis im Methanol-Synthesegasproduktstrom 2 wird bevorzugt laufend bzw. wiederholt gemessen (Istwert), wobei das besagte dritte Ventil V3, über das der erste Synthesegasteilstrom 51 in den Wassergas-Shift-Reaktor 100 gegeben wird, so geregelt wird, dass durch die im Wassergas-Shift-Reaktor 100 stattfindende Konvertierung von CO und H₂O zu H₂ und CO₂ das besagte Verhältnis an den vordefinierten Referenzwert im Bereich von 2,0 bis 2,1 angenähert wird.

In der Aminwascheinheit 102 wird eine kombinierte CO₂-Entfernung durchgeführt (aMDEA-Wäsche), wobei sowohl der konvertierte erste Synthesegasstrom (AOG-Strom) 51 als auch der besagte eine Teil 52a des ungeshifteten zweiten Synthesegasstromes 52 je einer Waschsäule zur CO₂-Reduzierung zugeführt wird. Während das CO₂ im konvertierten CO₂-reichen ersten Synthesegasstrom 51 vorzugsweise auf die für die Methanol-Synthese gewünschten etwa 3mol-% CO₂ reduziert wird, wird das CO₂ des besagten Teils 52a des unkonvertierten zweiten Synthesegasstromes 52 nahezu vollständig bis auf wenige mol-ppm entfernt. Neben den beiden waschmittelseitig energieeffizient miteinander verschalteten Waschsäulen kann insbesondere eine gemeinsame Regenerierung der Waschmittel der beiden Waschkolonnen in nur einer Regenerierkolonne durchgeführt werden.

Zur kompletten Entfernung der Komponenten Wasser und CO₂ wird der Teil 52a des unkonvertierten zweiten Synthesegasstromes 52 aus der CO₂-Wäsche 102 über eine erste Zuleitung 103 in eine Temperaturwechseladsorptionseinheit (TWA) 104 geführt, bevor es über eine zweite Zuleitung 105 aus der TWA 104 in eine kryogene Gastrennung gelangt, die in einer Cold Box 109 durchgeführt wird. Damit wird ein Ausfrieren dieser beiden Komponenten in der Cold Box 109 sowie etwaige Blockaden von Plattenwärmetauscher-Passagen, Produktreduktion sowie -ausfall verhindert.

Der beladene Adsorber der Temperaturwechseladsorptionseinheit 104 wird nach einer bestimmten Schrittfolge (Adsorbersequenz) mit einem Rohwasserstoffstrom 6a aus der besagten Cold Box 109 bei höheren Temperaturen regeneriert, bevor er erneut für die Adsorption zur Verfügung steht. Bei Bedarf, z.B. im Teillastfall, kann ein Teil des getrockneten Teils 52a des unkonvertierten zweiten Synthesegasteilstromes 52, der aus der Temperaturwechseladsorptionseinheit 104 kommt, dem zum Regenerieren/Spülen verwendeten Rohwasserstoffstrom 6a zugemischt werden, um die notwendige Regeneriergasmenge zur Verfügung zu stellen, und zwar über einen Bypass bzw. eine erste Überbrückungsleitung a, die von der zweiten Zuleitung 105 abzweigt.

Für den eingangs definierten, eingesetzten Synthesegasstrom (Acetylen-Offgas gemäß Tabelle) 5 wird vorzugsweise eine Methanwäsche 109 als kryogener Gastrennprozess durchgeführt. Bei einem niedrigerem H₂/CO-Verhältnis und höherem Druck im eingesetzten Synthesegasstrom (AOG) 5 kann aber auch ein Kondensationsprozess verwendet werden. Das Synthesegas 52a wird bei beiden Prozessen in den reinen CO-Produktstrom 4, den Rohwasserstoffstrom 6 und einem Restgasstrom 7 rektifikativ zerlegt.

Im Einzelnen kann bei der Methanwäsche 109 zunächst der getrocknete und von CO₂ befreite Teil 52a des zweiten (nicht-konvertierten) Synthesegasteilstroms 52 (insbesondere in Form eines zweiphasigen Gemisches) in einer Methanwaschsäule mit flüssigem Methan beaufschlagt werden, so dass CO aus der aufsteigenden gasförmigen H₂-reichen Phase ausgewaschen wird, wobei am Kopf der Methanwaschsäule der Rohwasserstoffstrom 6 abgezogen wird, der (über die Rohwasserstoffzuleitung 111) in die Druckwechseladsorptionsanlage 112 gegeben wird. Das Kondensat und das mit CO beladene Waschmethan, Stickstoff und noch gelöster Wasserstoff werden aus dem Sumpf der Methanwaschsäule abgezogen und in eine zweite Kolonne gegeben, wo ausgestrippter H₂ mit geringen Mengen an CO sowie N₂ über Kopf als Restgasstrom 7 abgezogen werden, und wobei die im Sumpf der zweiten Kolonne anfallende flüssige Phase in eine dritte Kolonne gegeben wird, aus der als Sumpfprodukt Methan gewonnen wird, das zurück in die Methanwaschsäule und in die H₂-Strippkolonne gegeben werden kann, wobei überschüssiges Methan dem Restgasstrom 7 zugegeben werden kann. Aus dem Kopf der dritten Kolonne wird eine CO und N₂ enthaltende Fraktion abgezogen, die in eine vierte Kolonne gegeben wird, aus deren Kopf im Wesentlichen N₂, das dem Restgasstrom 7 zugegeben wird, abgezogen wird, und aus deren Sumpf der CO-Produktstrom abgezogen wird. Anders ausgestaltete Methanwaschverfahren sind natürlich ebenfalls denkbar.

Das so erzeugte CO wird dann in einem CO-Kompressor 110 einerseits auf den oder die Produktdrücke, andererseits auf die notwendigen CO-Kreislaufdrücke verdichtet. Die CO-Kreislaufströme 4a werden in den vorgenannten Tieftemperaturprozess 109 zurückgeführt und dienen z.B. als Kolonnenheizung, sie liefern durch Entspannung aber auch Kälte (Joule-Thomson-Effekt) z.B. für Kopfkondensatoren. Der größte Teil des Kältebedarfs wird von einem CO-Expander 108 erzeugt, der einen CO-Teilstrom des CO-Kreislaufstromes 4a entspannt.

Der in der Cold Box 109 erzeugte Rohwasserstoff 6 wird zuerst größtenteils als Regenerier-/Spülgas (Rohwasserstoffstrom 6a über Spülgaszuleitung 106) in der Temperaturwechseladsorptionseinheit (TWA) 104 verwendet und dann (über die Regenerier-/Spülgasrückleitung 107 und die Rohwasserstoffzuleitung 111) zur Druckwechseladsorptionsanlage (DWA) 112 zur Feinreinigung geleitet.
Das Restgas 7 aus der Cold Box 109 wird vorzugsweise unter niedrigem Druck an der Anlagengrenze abgegeben und kann z.B. unterfeuert werden.

Um die gewünschte Wasserstoffproduktmenge zu erzeugen, werden der Rohwasserstoffstrom 6 aus der Cold Box 109 und der über das zweite Ventil V2 geleitete andere Teil 51b des konvertierten ersten Synthesegasstromes 51 zur DWA 112 geleitet, wo ein hochreiner H₂-Produktstrom (H₂>99,9 mol%) erzeugt wird. Bei Bedarf kann auch ein Teil des gewaschenen Teils 52a des unkonvertierten zweiten Synthesegasteilstromes 52 dem DWA-Einsatzgas 8 (Rohwasserstoffzuleitung 111) über eine zweite Überbrückungsleitung (Bypass) b zugeleitet werden (insbesondere bei geringer CO- und großer H₂-Produktmenge), die von der ersten Zuleitung 103 stromab der Aminwascheinheit 102 und stromauf der Temperaturwechseladsorptionseinheit 104 abzweigt und in die Rohwasserstoffzuleitung 111 mündet.

Umgekehrt kann insbesondere bei geringer H₂- und großer CO-Produktmenge ein Teil des Rohwasserstoffstromes 6 aus der Cold Box 109 über eine dritte Überbrückungsleitung (Bypass) c direkt in den Methanol-Synthesegasproduktstrom 2 (Ableitung 114) gefahren werden.

Es gibt demnach minimal eine und maximal drei Quellen zur H₂-Produktion, nämlich Rohwasserstoff 6 aus der Cold Box 109, konvertiertes Synthesegas 51b (über das zweite Ventil V2) sowie nichtkonvertiertes Synthesegas 52a (über Bypass b).

Da das Restgas 9 aus der Druckwechseladsorptionseinheit 112 hauptsächlich H₂ und auch CO enthält, wird es in einem Restgasverdichter 113 auf den gewünschten Druck verdichtet und bildet eine von maximal vier Komponenten des Methanol-Synthesegasproduktstromes 2. Die weiteren Bestandteile sind unkonvertiertes 52b und konvertiertes Synthesegas 51a und ggf. Rohwasserstoff 6 (über Bypass c). Mit Hilfe der Konvertierung kann für verschiedene vorgegebene CO- und H2-Produktmengen jeweils das notwendige Verhältnis (H₂-CO₂)/(CO+CO₂) von 2,0 bis 2,1 im Methanol-Synthesegasproduktstrom 2 eingestellt werden (drittes Ventil V3).

Das erfindungsgemäße Verfahren ermöglicht im Ergebnis insbesondere die Verwendung eines bisher kaum zur weiteren Wertstoffgewinnung genutzten, preiswert in großen Mengen als Nebenprodukt anfallenden Einsatzgases (AOG) zur Herstellung der qualitativ hochwertigen Produkte CO, H₂ und Methanol-Syngas.

Dabei kann insbesondere durch geeignete Kombination und Regelung der Prozessgruppen (Gesamtverschaltung) sowohl die CO- und H₂-Produktmenge als auch das für die Methanol-Synthese notwendige Verhältnis (H₂-CO₂)/(CO+CO₂) von 2,0 bis 2,1 eingestellt werden. Damit werden maximale Flexibilität für die drei Produkte in Abhängigkeit der zur Verfügung stehenden AOG-Menge und maximale Nutzung des AOG für diese Produkte erreicht, während nur ein geringer Restgasstrom 7 entsteht.

Schließlich kann insbesondere durch eine geeignete Verschaltung der Prozessströme in nur einer CO₂-Wascheinheit 102 sowohl das unkonvertierte 52a als auch das konvertierte AOG 51 behandelt werden und das Waschmittel in einer einzigen Kolonne regeneriert werden.

**Bezugszeichenliste**

| | |
|---|---|
| 1 | Anlage |
| 2 | Methanol-Synthesegasproduktstrom |
| 3 | H2-Produktstrom |
| 4 | CO-Produktstrom |
| 4a | CO-Teilstrom (Kreislauf) |
| 5 | Einsatz (Synthesegasstrom bzw. AOG-Strom) |
| 6 | Rohwasserstoffstrom |
| 6a, 6b | Rohwasserstoffstrom (zum/vom TWA-Regenerieren) |
| 7 | Restgasstrom aus Cold Box |
| 8 | DWA-Einsatzstrom |
| 9 | Restgasstrom (DWA) |
| 51 | erster Synthesegasteilstrom, konvertiert |
| 51a | Teil des ersten Synthesegasteilstroms |
| 51 b | Anderer Teil des ersten Synthesegasteilstroms |
| 52 | Zweiter Synthesegasteilstrom, unkonvertiert |
| 52a | Teil des zweiten Synthesegasteilstroms (zur/von CO2-Wäsche) |
| 52b | Anderer Teil des zweiten Synthesegasteilstroms (zum MeOH-Syngas) |
| 99 | Einsatz-Verdichter |
| 100 | Wassergas-Shift-Reaktor |
| 101 | Reinigungseinheit zur Reinigung des Einsatzgases (z.B. katalyt. Hydrierung) |
| 102 | CO2-Wäsche (z.B. Amin-Wäsche) |
| 103 | Erste Zuleitung |
| 104 | Temperaturwechseladsorptionseinheit (TWA) |
| 105 | Zweite Zuleitung |
| 106 | Regenerier-/Spülgaszuleitung |
| 107 | Regenerier-/Spülgasrückleitung |
| 108 | CO-Expander |
| 109 | Methanwascheinheit (Cold Box) |
| 110 | CO-Verdichter |
| 111 | Rohwasserstoffzuleitung zu DWA |
| 112 | Druckwechseladsorptionseinheit (DWA) |
| 113 | DWA-Restgasverdichter |
| 114 | Ableitung für Methanol-Synthesegasprodukt |
| V1, V2, V3 | Ventile |
| a, b, c | Überbrückungsleitungen (Bypässe) |

## Patentansprüche

1. Verfahren zur Herstellung eines Methanol-Synthesegasproduktstromes (2), eines H₂-Produktstromes (3) und eines CO-Produktstromes (4) aus einem H₂ und CO enthaltenden Synthesegasstrom (5), aufweisend die Schritte:
- Aufteilen des Synthesegasstromes (5) in einen ersten und einen zweiten Synthesegasteilstrom (51, 52), wobei lediglich im ersten Synthesegasteilstrom (51) enthaltenes CO mit dem ersten Synthesegasteilstrom (51) zugemischtem Wasserdampf zu CO₂ und H₂ konvertiert wird (100), wobei
- der erste Synthesegasteilstrom (51) und ein Teil (52a) des zweiten Synthesegasteilstromes (52) jeweils in einer separaten Kolonne zum Auswaschen von CO₂ mit einem aminhaltigen Waschmittel gewaschen werden (102), wobei das Waschmittel in einer gemeinsamen Kolonne regeneriert wird,
- wobei der Methanol-Synthesegasproduktstrom (2) aus einem Teil (51a) des gewaschenen, konvertierten ersten Synthesegasteilstromes (51) und/oder dem anderen Teil (52b) des unkonvertierten, zweiten Synthesegasstromes (52) gebildet wird, so dass ein für die Methanol-Synthese notwendiges Verhältnis von (H₂-CO₂)/(CO+CO₂) im Methanol-Synthesegasproduktstrom (2) in einem Bereich von 2,0 bis 2,1 eingestellt wird, wobei
- der gewaschene eine Teil (52a) des zweiten nicht-konvertierten Synthesegasteilstromes (52) zur Herstellung des CO-Produktstromes (4) und des H₂-Produktstromes (3) verwendet wird, und wobei
- der andere Teil (51b) des gewaschenen, konvertierten ersten Synthesegasteilstromes (51) zur Herstellung des H₂-Produktstromes (3) verwendet wird.

2. Verfahren nach Anspruch 1, **dadurch gekennzeichnet, dass** der Synthesegasstrom (5) ein in einer Acetylenanlage erzeugter Acetylen-Offgasstrom ist.

3. Verfahren nach Anspruch 1 oder 2, **dadurch gekennzeichnet, dass** der Synthesegasstrom (5) vor dem besagten Aufteilen auf einen Druck im Bereich von 25 bar bis 40 bar verdichtet wird (99).

4. Verfahren nach Anspruch 2, **dadurch gekennzeichnet, dass** im Synthesegasstrom (5) enthaltene, ungesättigte Kohlenwasserstoffe nach dem Verdichten und vor dem Aufteilen des Synthesegasstromes (5) zu gesättigten Kohlenwasserstoffen katalytisch hydriert werden (101), wobei C₂H₂ und/oder C₂H₄ zu C₂H₆ sowie C₃H₄ und/oder C₃H₆ zu C₃H₈ hydriert wird, und wobei im Synthesegasstrom (5) enthaltener Sauerstoff mit ebenfalls enthaltenem H₂ und CO zu H₂O und CO₂ umgesetzt wird, und wobei zusätzlich Schwefel- und NMP-Spuren aus dem Synthesegasstrom (5) entfernt werden.

5. Verfahren nach einem der vorhergehenden Ansprüche, **dadurch gekennzeichnet, dass** der eine Teil (52a) des zweiten Synthesegasteilstroms (52) nach einer CO₂-Wäsche zum Trocknen und zur Entfernung von CO₂ einer Temperaturwechseladsorption (104) unterzogen wird, wobei zumindest ein Adsorber im besagten Teil (52a) des zweiten Synthesegasteilstromes (52) enthaltenes H₂O und CO₂ bei einer niedrigen Temperatur adsorbiert, und der mit den adsorbierten Komponenten beladene mindestens eine Adsorber bei einer höheren Temperatur durch Spülen mit einem Rohwasserstoffstrom (6a) regeneriert wird, wobei dem Rohwasserstoffstrom (6a) ein Teil des durch die besagte Temperaturwechseladsorption (104) getrockneten und von CO₂ befreiten Teils (52a) des zweiten Synthesegasteilstroms (52) zugemischt wird (a).

6. Verfahren nach Anspruch 5, **dadurch gekennzeichnet, dass** der getrocknete und von CO₂ befreite eine Teil (52a) des zweiten Synthesegasteilstroms (52) in einer Cold Box (109) zumindest in den CO-Produktstrom (4) und einen Rohwasserstoffstrom (6) sowie einen Restgasstrom (7) getrennt wird, wobei der CO-Produktstrom (4) verdichtet wird (110), und wobei zumindest ein verdichteter Teilstrom (4a) des CO-Produktstroms (4) zur Erzeugung der für die besagte Trennung benötigten Kälte und/oder Wärme verwendet wird, und wobei der Restgasstrom (7) an einer Anlagengrenze abgegeben und/oder verfeuert wird.

7. Verfahren nach den Ansprüchen 5 und 6, **dadurch gekennzeichnet, dass** der Rohwasserstoffstrom (6a) zum Regenerieren des mindestens einen Adsorbers ein Teilstrom des Rohwasserstoffstromes (6) aus der Cold Box (109) ist.

8. Verfahren nach Anspruch 6 oder 7, **dadurch gekennzeichnet, dass** der Rohwasserstoffstrom (6) und der besagte andere Teil (51b) des gewaschenen, konvertierten ersten Synthesegasteilstromes (51) zu einem wasserstoffreichen Einsatzstoffstrom (8) vermischt werden, der zum Erzeugen des H₂-Produktstromes (3) einer Druckwechseladsorption (112) unterzogen wird, wobei im Einsatzstoffstrom (8) enthaltener Wasserstoff unter hohem Druck zumindest einen Adsorber passiert und den besagten H₂-Produktstrom (3) bildet und im Einsatzstoffstrom (8) enthaltene schwerere Komponenten am mindestens einen Adsorber adsorbiert werden, und wobei die am mindestens einen Adsorber adsorbierten Komponenten bei niedrigerem Druck desorbiert und mit einem aus einem Teilstrom des erzeugten H₂-Produktstromes (3) gebildeten Spülgas gespült werden, wobei ein die desorbierten Komponenten sowie das Spülgas enthaltener Restgastrom (9) verdichtet (113) und dem Methanol-Synthesegasstrom (2) zugegeben wird.

9. Verfahren nach Anspruch 8, **dadurch gekennzeichnet, dass** ein Teil des gewaschenen Teils (52a) des zweiten, nicht-konvertierten Synthesegasteilstromes (52) dem Einsatzstoffstrom (8) für die Druckwechseladsorptionseinheit (112) zugemischt wird (b).

10. Verfahren nach Anspruch 8 oder 9, **dadurch gekennzeichnet, dass** ein Teil des Rohwasserstoffstromes (6) in den Methanol-Synthesegasproduktstrom (2) gegeben wird (c).

## Claims

1. Method for production of a methanol-synthesis gas product stream (2), an H₂ product stream (3) and a CO product stream (4) from an H₂- and CO-containing synthesis gas stream (5), having the steps:
- portioning the synthesis gas stream (5) into a first and a second synthesis gas substream (51, 52), wherein only CO contained in the first synthesis gas substream (51) is converted (100) to CO₂ and H₂ using steam admixed to the first synthesis gas substream (51), wherein
- the first synthesis gas substream (51) and a part (52a) of the second synthesis gas substream (52) are scrubbed (102), each in a separate column, with an amine-containing scrubbing medium, for scrubbing out CO₂, wherein the scrubbing medium is regenerated in a shared column,
- wherein the methanol-synthesis gas product stream (2) is formed from one part (51a) of the scrubbed converted first synthesis gas substream (51) and/or the other part (52b) of the unconverted second synthesis gas stream (52), in such a manner that a ratio of (H₂-CO₂)/(CO+CO₂) that is required for the methanol synthesis is established in the methanol-synthesis gas product stream (2) in a range from 2.0 to 2.1, wherein
- the scrubbed one part (52a) of the second non-converted synthesis gas substream (52) is used for production of the CO product stream (4) and the H₂ product stream (3), and wherein
- the other part (51b) of the scrubbed converted first synthesis gas substream (51) is used for production of the H₂ product stream (3).

2. Method according to Claim 1, **characterized in that** the synthesis gas stream (5) is an acetylene offgas stream produced in an acetylene plant.

3. Method according to Claim 1 or 2, **characterized in that** the synthesis gas stream (5), before the said division, is compressed (99) to a pressure in the range from 25 bar to 40 bar.

4. Method according to Claim 2, **characterized in that** unsaturated hydrocarbons that are present in the synthesis gas stream (5) are catalytically hydrogenated (101) to form saturated hydrocarbons after the compression and before the division of the synthesis gas stream (5), wherein C₂H₂ and/or C₂H₄ is hydrogenated to form C₂H₆ and also C₃H₄ and/or C₃H₆ is hydrogenated to form C₃H₈, and wherein oxygen present in the synthesis gas stream (5) is reacted with H₂ and CO that are likewise present to form H₂O and CO₂, and wherein in addition traces of sulphur and NMP are removed from the synthesis gas stream (5).

5. Method according to any one of the preceding claims, **characterized in that** the one part (52a) of the second synthesis gas substream (52), after a CO₂ scrubbing for drying and for removing CO₂, is subjected to a temperature-swing adsorption (104), wherein at least one adsorber adsorbs, at a low temperature, H₂O and CO₂ present in the said part (52a) of the second synthesis gas substream (52), and the at least one adsorber loaded with the adsorbed components is regenerated at a higher temperature by purging with a crude hydrogen stream (6a), wherein a part of the part (52a) of the second synthesis gas substream (52) that is dried by the said temperature-swing adsorption (104) and is freed from CO₂ is admixed (a) to the crude hydrogen stream (6a).

6. Method according to Claim 5, **characterized in that** the one part (52a) of the second synthesis gas substream (52) that is dried and freed from CO₂ is separated, in a cold box (109), at least into the CO product stream (4) and a crude hydrogen stream (6), and also a residual gas stream (7), wherein the CO product stream (4) is compressed (110), and wherein at least one compressed substream (4a) of the CO product stream (4) is used for generating the cold and/or heat required for the said separation, and wherein the residual gas stream (7) is delivered at a battery limit and/or burnt.

7. Method according to Claims 5 and 6, **characterized in that** the crude hydrogen stream (6a) for regenerating the at least one adsorber is a substream of the crude hydrogen stream (6) from the cold box (109).

8. Method according to Claim 6 or 7, **characterized in that** the crude hydrogen stream (6) and the said other part (51b) of the scrubbed converted first synthesis gas substream (51) are mixed to form a hydrogen-rich feed stream (8) which is subjected to a pressure-swing adsorption (112) to produce the H₂ product stream (3), wherein hydrogen present in the feed stream (8) is passed under high pressure through at least one adsorber and forms the said H₂ product stream (3), and heavier components present in the feed stream (8) are adsorbed on the at least one adsorber, and wherein the components that are adsorbed on at least one adsorber are desorbed at lower pressure, and are purged with a purge gas formed from a substream of the H₂ product stream (3) produced, wherein a residual gas stream (9) containing the desorbed components and also the purge gas is compressed (113) and fed to the methanol-synthesis gas stream (2) .

9. Method according to Claim 8, **characterized in that** a part of the scrubbed part (52a) of the second, non-converted, synthesis gas substream (52) is admixed (b) to the feed stream (8) for the pressure-swing adsorption unit (112).

10. Method according to Claim 8 or 9, **characterized in that** a part of the crude hydrogen stream (6) is added (c) to the methanol-synthesis gas product stream (2).

## Revendications

1. Procédé de fabrication d'un courant de produit de gaz de synthèse de méthanol (2), d'un courant de produit d'H₂ (3) et d'un courant de produit de CO (4) à partir d'un courant de gaz de synthèse (5) contenant de l' H₂ et du CO, comprenant les étapes suivantes :
- la division du courant de gaz de synthèse (5) en un premier et un deuxième courant partiel de gaz de synthèse (51, 52), uniquement le CO contenu dans le premier courant partiel de gaz de synthèse (51) étant converti (100) avec de la vapeur d'eau incorporée dans le premier courant partiel de gaz de synthèse (51) pour former du CO₂ et de l'H₂,
- le premier courant partiel de gaz de synthèse (51) et une partie (52a) du deuxième courant partiel de gaz de synthèse (52) étant chacun lavés (102) dans une colonne séparée pour l'élimination par lavage du CO₂ avec un agent de lavage contenant des amines, l'agent de lavage étant régénéré dans une colonne commune,
- le courant de produit de gaz de synthèse de méthanol (2) étant formé à partir d'une partie (51a) du premier courant partiel de gaz de synthèse (51) converti lavé et/ou de l'autre partie (52b) du deuxième courant de gaz de synthèse (52) non converti, de telle sorte qu'un rapport (H₂-CO₂)/(CO+CO₂) nécessaire pour la synthèse de méthanol dans le courant de produit de gaz de synthèse de méthanol (2) dans une plage allant de 2,0 à 2,1 soit ajusté,
- la partie lavée (52a) du deuxième courant partiel de gaz de synthèse non converti (52) étant utilisée pour la fabrication du courant de produit de CO (4) et du courant de produit d'H₂ (3), et
- l'autre partie (51b) du premier courant partiel de gaz de synthèse converti lavé (51) étant utilisée pour la fabrication du courant de produit d'H₂ (3).

2. Procédé selon la revendication 1, **caractérisé en ce que** le courant de gaz de synthèse (5) est un courant de gaz d'échappement d'acétylène formé dans une unité de production d'acétylène.

3. Procédé selon la revendication 1 ou 2, **caractérisé en ce que** le courant de gaz de synthèse (5) est comprimé (99) à une pression dans la plage allant de 25 bar à 40 bar avant ladite division.

4. Procédé selon la revendication 2, **caractérisé en ce que** les hydrocarbures insaturés contenus dans le courant de gaz de synthèse (5) sont hydrogénés catalytiquement (101) en hydrocarbures saturés après la compression et avant la division du courant de gaz de synthèse (5), C₂H₂ et/ou C₂H₄ étant hydrogénés en C₂H₆ et C₃H₄ et/ou C₃H₆ en C₃H₈, et l'oxygène contenu dans le courant de gaz de synthèse (5) étant mis en réaction avec l'H₂ et le CO également contenus pour former de l'H₂O et du CO₂, et des traces de soufre et de NMP étant en outre éliminées du courant de gaz de synthèse (5).

5. Procédé selon l'une quelconque des revendications précédentes, **caractérisé en ce que** la partie (52a) du deuxième courant partiel de gaz de synthèse (52) est soumise après un lavage au CO₂ à une adsorption modulée en température (104) pour le séchage et l'élimination du CO₂, au moins un adsorbeur adsorbant l'H₂O et le CO₂ contenus dans ladite partie (52a) du deuxième courant partiel de gaz de synthèse (52) à une température basse, et ledit au moins un adsorbeur chargé avec les composants adsorbés étant régénéré à une température plus élevée par rinçage avec un courant d'hydrogène brut (6a), une partie de la partie (52a) du deuxième courant partiel de gaz de synthèse (52) séchée par ladite adsorption modulée en température (104) et débarrassée du CO₂ étant incorporée (a) dans le courant d'hydrogène brut (6a).

6. Procédé selon la revendication 5, **caractérisé en ce que** la partie (52a) du deuxième courant partiel de gaz de synthèse (52) séchée et débarrassée du CO₂ est séparée dans une boîte froide (109) au moins en le courant de produit de CO (4) et un courant d'hydrogène brut (6), ainsi qu'un courant de gaz résiduel (7), le courant de produit de CO (4) étant comprimé (110) et au moins un courant partiel comprimé (4a) du courant de produit de CO (4) étant utilisé pour la formation du froid et/ou de la chaleur nécessaires pour ladite séparation, et le courant de gaz résiduel (7) étant déchargé et/ou brûlé à une limite de l'unité.

7. Procédé selon les revendications 5 et 6, **caractérisé en ce que** le courant d'hydrogène brut (6a) pour la régénération dudit au moins un adsorbeur est un courant partiel du courant d'hydrogène brut (6) issu de la boîte froide (109).

8. Procédé selon la revendication 6 ou 7, **caractérisé en ce que** le courant d'hydrogène brut (6) et ladite autre partie (51b) du premier courant partiel de gaz de synthèse converti lavé (51) sont mélangés pour former un courant de charge riche en hydrogène (8), qui est soumis à une adsorption modulée en pression (112) pour former le courant de produit d'H₂ (3), l'hydrogène contenu dans le courant de charge (8) passant dans au moins un adsorbeur à pression élevée et formant ledit courant de produit d'H₂ (3), et les composants plus lourds contenus dans le courant de charge (8) étant adsorbés sur ledit au moins un adsorbeur, et les composants adsorbés sur ledit au moins un adsorbeur étant désorbés à pression plus basse et rincés avec un gaz de rinçage formé par un courant partiel du courant de produit d'H₂ (3) formé, un courant de gaz résiduel (9) contenant les composants désorbés et le gaz de rinçage étant comprimé (113) et introduit dans le courant de gaz de synthèse de méthanol (2) .

9. Procédé selon la revendication 8, **caractérisé en ce qu'**une partie de la partie lavée (52a) du deuxième courant partiel de gaz de synthèse non converti (52) est incorporée (b) dans le courant de charge (8) pour l'unité d'adsorption modulée en pression (112).

10. Procédé selon la revendication 8 ou 9, **caractérisé en ce qu'**une partie du courant d'hydrogène brut (6) est introduite (c) dans le courant de produit de gaz de synthèse de méthanol (2).
